# EUROPEAN PATENT SPECIFICATION

(11) **EP 2 644 219 B1**
(45) Date of publication and mention of the grant of the patent: **06.01.2016**
(21) Application number: 13475501.6
(22) Date of filing: 28.03.2013
(51) Int. Cl.: A61M 5/32

(54) **Safety disposable needle or cannula**
Einwegsicherheitsnadel oder -kanüle
Aiguille ou canule jetable de sécurité

(30) Priority: 28.03.2012 SK 500122012
(43) Date of publication of application: 02.10.2013
(73) Proprietor: Chiranat T. Injecta, a.s., 91601 Stara Tura (SK); Buday, Marian, 95144 Vycapy-Opatovce (SK)
(72) Inventor: Buday, Marián, 95144 Výcapy-Opatovce (SK)
(74) Representative: Belicka, Ivan

(56) References cited:
- EP-A1- 2 090 326
- US-A- 4 892 521
- US-A- 5 295 975
- US-A- 5 360 408
- US-A- 5 591 138
- US-A1- 2005 113 750
- US-A1- 2009 024 093

## Description

### Scope of Technology

This technical solution deals with a safety disposable needle or cannula, which becomes unusable after being used by pressing the lever mechanism.

### Background Art

In the present, quite a large amount of disposable needles, cannulas or needles for single use are known. The Slovak patent application PP161-97 describes a syringe with a retractable needle, which has a needle moving from open position to retracted position inside the cylinder, and the handle is equipped with a compression spring to push the needle into retracted position. The needle is placed in a sleeve placed in the handle, which contains a ring placed in the groove of the sleeve, to which the floating case on the sleeve fits tightly, which when moving forward pulls the ring out of the groove and so enables the needle with the sleeve to click inside the piston by the action of the spring. In another Slovak patent application PP 533-96 a syringe is described, which consists of a cylinder with a piston, at the front end of the cylinder there is a sliding element to which a needle is attached. Behind the sliding element there is the sealing of the chamber and a stop. When the piston moves forward during injection, the lower end of the piston contacts the top surface of the stop, which leads to disruption of the circular part, rests of the stop break away from the sliding element, which is then drawn into the chamber of the piston by action of the spring with such force, that the sliding element is kept seized in this part. Then the syringe cannot be reused. The application CZ 3628-2001 describes a disposable syringe, so called safety syringe, containing a cylindrical body of piston pump of the syringe, in its extension there is a needle, which is inextricably connected with the body containing the piston. The body of the piston pump contains a proximal part with a needle and a distal part with a larger diameter, which contains a piston, wherein the proximal part contains closing means. The application CZ 3167-1996 describes a syringe with a retractable needle for single use, consisting of a cylindrical body with entry from one side of the slide, a movable middle and the other side for insertion of the hub with a needle, protective cap, middle with a rounded projection to capture the hub with the needle and a seal in the three-part structure. Some of the existing solutions in use provide safety in the sense that after using the sharp tip of the needle hides. Hiding the tip prevents accidental contact of the needle tip with other live but also inanimate objects. US 2009/0024093 discloses a safety needle in accordance with the preamble of claim 1. US 5 360408 discloses an alternative safety needle.

### Fundamentals of the technical solution

Fundamentals of the present technical solution according to claim 1 is that the blunt end of the needle or cannula is connected with the hollow attachable portion through the insulation bed, which has a neck at the end for attaching to the syringe, the hollow attachable part has a shape of a double tube with a blinded bottom, and this bottom is equipped with a groove, in which a compression spring is fixed, the outer tube of the attachable part is provided with at least two shoulders, the length of the inner tube of the attachable part does not exceed the outer tube and contains the inner tube of the cover with a conical opening for guiding the needle or cannula, and the cover has a shape corresponding to the shape of the hollow attachable part, where the cover is upheld to the hollow attachable part by at least one snap element with a blocking element, then the cover is provided with a groove on the inside part, wherein the other end of the compression spring is fastened in this groove, a front element with an opening is attached from the outside of the cover using snap elements, wherein the front element is provided with a flexible element on the internal shoulder to cover the opening after removing the needle or cannula out of it, the blocking element is controlled by a lever mechanism situated at the outer side of the hollow attachable part. The compression spring may have conical shape and its wider end may be fixed in a groove of the attachable part and the narrower end of the spring is fixed in the groove of the cover. After using the disposable needle or canulla, after pushing the lever mechanism the blocking element is pulled out of the shoulder and the snap element is released. The compression spring removes the cover away from attachable part to such a distance, that the tip of the needle or cannula is brought in front of the internal shoulder and the flexible element, which covers the opening in the inner shoulder and prevents redeploying the needle or cannula through the opening in the inner shoulder and thereby prevents re-using the needle or cannula. The attachable part and the cover are held together by the compression spring tightly attached to these two parts.

### Description of the drawings

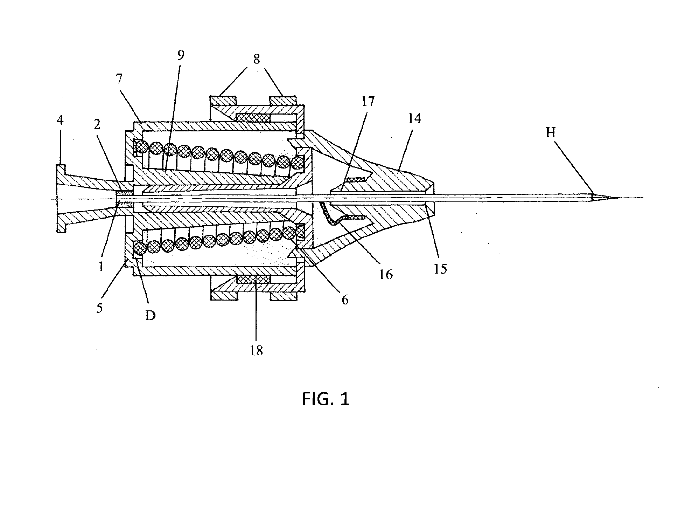

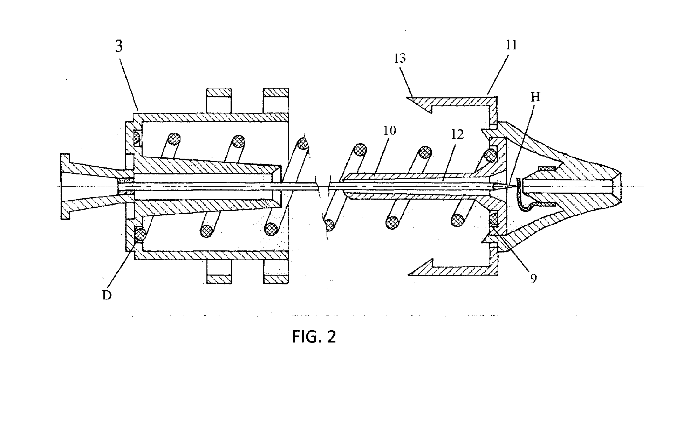

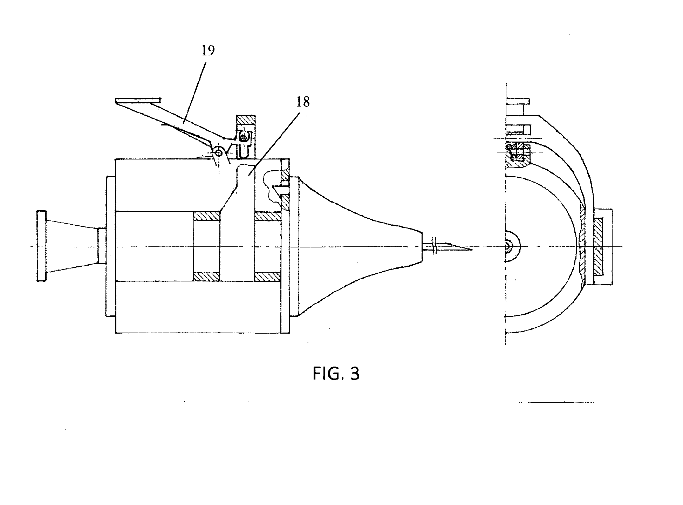

Fig. No. 1, No. 2 and No. 3 show the safety disposable needle or cannula in the cut and partial cut view.

### Examples of embodiments

Blunt end 1 of the safety disposable needle or cannula is attached to the hollow attachable part 3 by the isolation bed 2, which has a neck 4 at its end to attach to syringe, the hollow attachable part 3 has the shape of a double tube with a blinded bottom 5, and this bottom 5 is equipped with a groove D in which the compression spring 6 is fixed, the outer tube 7 of the attachable part 3 is provided with at least two shoulders 8, the length of the inner tube 9 of the attachable part 3 does not exceed the outer tube 7 and it contains the inner tube 10 of cover 11 with an internal conical opening 12 for guiding the needle or cannula, wherein the cover 11 has a shape corresponding to the shape of the hollow attachable part 3, where the cover 11 is upheld to the hollow attachable part 3 by at least one snap element 13 with a blocking element 18, then the inside of the cover 11 is provided with the groove d, wherein in this groove d the other end of the compression spring 6 is fixed, the front element 14 with the opening 15 is by means of snap elements 12 fixed on the outside of the cover 11, wherein the front element is provided with flexible element 16 on the internal shoulder 17 to cover the opening 15 after removing the needle or cannula from it, the blocking element 18 is controlled by a lever mechanism 19 situated at the outer side of the hollow attachable part 3. The compression spring 6 can have conical shape with the fact that the wider end of the spring 6 is fixed in groove D of the attachable part 3 and the narrower end of the spring 6 is fixed in groove d of the cover 11. After using the disposable needle or cannula, by pressing the lever mechanism 19 the blocking element 18 is pulled out of the shoulder 8, thereby releasing the snap element 13. The compression spring 6 removes the cover 11 from the attachable part 3 in a distance that the tip of the needle or cannula H is brought in front of the internal shoulder 17 and flexible element 16, which covers the opening in the inner shoulder 17 and prevents re-transfer of the needle or cannula through the opening in the inner shoulder 17 and thereby prevents re-use of the needle or cannula. The attachable part 3 and the cover 11 are held together by the compression spring 6 firmly attached to these two parts.

## Claims

1. Safety disposable needle or canulla, wherein a blunt end (1) of a needle or cannula H is attached to a hollow attachable part (3) by an isolation bed (2), which has a neck (4) at its end to attach to a syringe, the hollow attachable part (3) has the shape of a double tube with a blinded bottom (5), and this bottom (5) is equipped with a groove D in which a compression spring (6) is fixed, an outer tube (7) of the attachable part (3) is provided with at least two shoulders (8) **characterized in that**, the length of an inner tube (9) of the attachable part (3) does not exceed the outer tube (7) and which contains an inner tube (10) of cover (11) with an internal conical opening (12) for guiding the needle or cannula, wherein the cover (11) has a shape corresponding to the shape of the hollow attachable part (3), where the cover (11) is upheld to the hollow attachable part (3) by at least one snap element (13) with a blocking element (18), then the inside of the cover (11) is provided with a groove D, d wherein in this groove D the other end of the compression spring (6) is fixed, a front element (14) with opening (15) is by means of snap elements (13) fixed on the outside of the cover (11), wherein the front element is provided with a flexible element (16) on an internal shoulder (17) to cover the opening (15) after removing the needle or cannula from it, the blocking element (18) is controlled by a lever mechanism (19) situated at the outer side of the hollow attachable part (3).

2. Safety disposable needle or canulla as in Claim 1, **characterised in that** the compression spring (6) has conical shape, the wider end of the needle (4) is fixed in the groove (D) of the attachable part (3) and the narrower end is fixed in the groove (d) of the cover (11).

## Patentansprüche

1. Sichere Einmalgebrauchsnadel oder Einmalgebrauchskanüle, **dadurch gekennzeichnet, dass** das stumpfe Ende (1) der Nadel oder Kanüle durch das Isolationsbett (2) mit dem Hohlansatzstück (3), das einen Hals (4) am Ende zur Befestigung an die Spritze hat, verbunden ist, der Hohlansatzstück (3) hat die Form eines Doppelrohres mit verblindetem Boden (5), wobei dieser Boden (5) mit einer Nut (D), in der eine Druckfeder (6) gelagert ist, versehen ist, das Außenrohr (7) des Ansatzstücks (3) ist mindestens mit zwei Schultern (8) versehen, die Länge des Innenrohrs (9) des Hohlansatzstücks (3), in dem das Innerohr (10) der Schutzkappe (11) mit konischer Innenöffnung (12) zur Führung der Nadel oder Kanüle gelagert ist, ist nicht größer als die Länge das Außenrohrs (7), wobei die Schutzkappe (11) eine Form hat, die der Form des Hohlansatzstücks (3) entspricht, wobei die Schutzkappe (11) an dem Hohlansatzstück (3) mindestens durch einen Rastteil (13) mit einem Sperrelement (18) gehalten wird, die Schutzkappe (11) ist auf der Innenseite mit einer Nut (d) versehen, wobei das andere Ende der Druckfeder (6) in dieser Nut (d) befestigt ist, von der Außenseite der Schutzkappe (11) ist mit Hilfe der Rastelemente (12) ein Vorderteil (14) mit einer Öffnung (15) befestigt, der am inneren Schulter (17) mit einem elastischen Element (16) zum Abdecken der Öffnung (15) beim Zurückziehen der Nadel oder Kanüle versehen ist, das Sperrelement (18) wird durch ein an der Außenseite des Hohlansatzstücks (3) angeordnetes Hebelmechanismus (19) gesteuert.

2. Sichere Einmalgebrauchsnadel oder Einmalgebrauchskanüle nach Anspruch 1, **dadurch gekennzeichnet, dass** die Druckfeder (6) eine konische Form hat und das breitere Ende der Feder (6) in der Nut (D) des Ansatzstücks (3) und das engere Ende der Feder (6) in der Nut (d) der Schutzkappe (11) befestigt ist.

## Revendications

1. L'aiguille de sécurité à usage unique ou la canule **caractérisée en ce que** l'extrémité émoussée (1) de l'aiguille ou de la canule est rattachée par un isolant (2) à la pièce creuse de fixation (3), qui a une gorge (4) à son extrémité pour être rattachée à une seringue, la pièce creuse de fixation (3) a la forme d'un tube double à fond aveugle (5) et ce fond (5) est équipé d'une rainure (D) dans laquelle est fixé un ressort de compression (6), le tube extérieur (7) de la pièce de fixation (3) est fourni avec deux embases (8) minimum, la longueur du tube intérieur (9) de la pièce de fixation (3) ne dépasse pas celle du tube extérieur (7) et il contient le tube intérieur (10) du capot (11) avec un orifice conique intérieur (12) pour guider l'aiguille ou la canule, le capot (11) ayant une forme correspondant à la forme de la pièce creuse de fixation (3) et le capot (11) est fixé à la pièce creuse de fixation (3) par au moins un élément de fermeture (13) avec un composant de blocage (18), puis le capot (11) est équipé sur sa face interne d'une rainure (d), tandis que l'autre extrémité du ressort de compression (6) est fixé dans la rainure (d), l'élément avant (14) avec un orifice (15) est équipé d'un élément flexible (16) sur l'embase intérieure (17) pour couvrir l'orifice après retrait de l'aiguille ou de la canule et est fixé à l'aide des éléments de fermeture (12) à l'extérieur du capot (11), l'élément de blocage (18) est contrôlé par un mécanisme de levier (19) situé sur la face externe de la pièce creuse de fixation (3).

2. L'aiguille de sécurité à usage unique ou la canule selon la revendication 1 caractérisée en ce fait que le ressort de compression (6) a une forme conique, l'extrémité plus large de l'aiguille (6) est fixée dans la rainure (D) de la pièce de fixation (3) et l'extrémité plus étroite (6) est fixée dans la rainure (d) du capot (11).
